# EUROPÄISCHE PATENTSCHRIFT

(11) **EP 1 663 930 B1**
(45) Veröffentlichungstag und Bekanntmachung des Hinweises auf die Patenterteilung: **30.07.2008**
(21) Anmeldenummer: 04764855.5
(22) Anmeldetag: 06.09.2004
(51) Int. Cl.: C07C 43/11, C08G 65/329, C11D 1/72

(54) **IN ALKALIEN STABILE ALKOXYLATE**
ALKOXYLATES THAT ARE STABLE IN ALKALIS
ALCOXYLATES STABLES EN ALCALIS

(30) Priorität: 10.09.2003 DE 10341724
(43) Veröffentlichungstag der Anmeldung: 07.06.2006
(73) Patentinhaber: BASF SE, 67056 Ludwigshafen (DE)
(72) Erfinder: NÖRENBERG, Ralf, 55218 Ingelheim (DE); ANNEN, Ulrich, 67454 Hassloch (DE)
(74) Vertreter: Isenbruck, Günter
(86) Internationale Anmeldenummer: PCT/EP2004/009904
(87) Internationale Veröffentlichungsnummer: WO 2005/026094

(56) Entgegenhaltungen:
- EP-A- 1 050 524
- DE-A1- 3 626 567
- US-A- 3 407 142
- US-A- 4 753 885
- US-A- 4 973 423
- US-A- 5 071 454
- US-A- 5 677 273
- T.P. HOBIN: "Model Polyethers I - Synthesis by the Williamson Reaction" POLYMER, Bd. 6, 1965, Seiten 403-409, XP002315295
- HIROATSU MATSUURA ET AL: "Raman Spectroscopic studies of Molecular Conformation of alpha-alkyl-omega-alkoxyoligo(oxyethylene) s" J. MOL. STRUCT., Bd. 189, 1988, Seiten 249-256, XP002315296
- YIYAN CHEN AND GREGORY L. BAKER: "Synthesis and Properties of ABA Amphiphiles" J. ORG. CHEM., Bd. 64, Nr. 18, 1999, Seiten 6870-6873, XP001070793

## Beschreibung

Die Erfindung betrifft ein Verfahren zur Herstellung von in Alkalien stabilen Alkoxylaten, deren Verwendung sowie die Alkoxylate enthaltende Formulierungen.

Die schnelle Benetzung von Oberflächen spielt in vielen Bereichen des täglichen Lebens und in vielen industriellen Prozessen, beispielsweise bei der Reinigung oder Beschichtung von Substraten, eine zentrale Rolle. In vielen Formulierungen werden deshalb unterschiedlich große Mengen von Alkoholen wie Ethanol oder Isopropanol eingesetzt, um beispielsweise die Grenzflächen- oder Oberflächenspannung zu erniedrigen und damit das Benetzungsvermögen der Formulierungen zu verbessern. Dabei ist es üblich, wässrigen Formulierungen oft größere Mengen dieser Alkohole beizumischen. Die physiologische Wirkung der Alkohole ist allerdings bedenklich, und die Exposition des Anwenders solcher Formulierungen ist durch den hohen Dampfdruck der Alkohole hoch. Daher sollten heute beispielsweise in Formulierungen, die vom Anwender direkt gehandhabt werden, nur geringe Mengen an Alkoholen oder kein Alkohol vorliegen. Für sehr schnell netzende Formulierungen, wie Feuchthaltemittel in der Druckindustrie oder Additive für Beschichtungsformulierungen, zum Beispiel Sprühlackierungen, stellen sie jedoch immer noch einen notwendigen Bestandteil dar.

Seit einigen Jahren ist die sehr gut netzende Wirkung von sehr hydrophoben, kompakten Alkoholen, die aus Acetylen und Aldehyden hergestellt werden können, bekannt. Es handelt sich dabei insbesondere um Dihydroxyalkine. Diese Produkte sind jedoch nicht mit allen Reinigungsmittelformulierungen verträglich und können oft nur mit Hilfe von Solubilisatoren wie Cumolsulfonat, Ethylenglykol etc. verwendet werden. Oft muss dabei eine größere Menge des Solubilisators im Vergleich zum Netzhilfsmittel eingesetzt werden, so dass sich hohe Folgekosten durch die Verwendung der Dihydroxyalkine ergeben. Zudem wird die Wirkung des Netzhilfsmittels bei der Abmischung mit größeren Mengen an Solubilisatoren verschlechtert.

Ein üblicher Weg, um die Benetzungsgeschwindigkeit von wässrigen Formulierungen zu erhöhen, besteht in der Verwendung von Tensiden, die sich an Grenzflächen anlagern und dabei die Grenzflächenspannung erniedrigen. Während durch Zumischen von Alkoholen wie Ethanol oder Isopropanol zu wässrigen Formulierungen das resultierende Wasser/Lösemittel-Gemisch eine gegenüber Wasser niedrigere Oberflächenspan nung aufweist und damit ein verbessertes Netzverhalten zeigt, ist die Benetzung oder Oberflächenbelegung bei Verwendung von Tensidsystemen zeitabhängig. Die Tensidmoleküle müssen zunächst an die Oberfläche diffundieren und dort einen Grenzflächenfilm aufbauen, wodurch die Grenzflächenspannung bzw. die Oberflächenspannung im Falle des Kontakts von Wasser und Luft sinkt. Bei sehr schnellen Prozessen wie etwa Sprüh- oder Netzprozessen, beispielsweise von Lacken in curtain coating-Prozessen, ist die Zeit, in der die Oberflächen- oder Grenzflächenspannung durch das Tensidsystem auf den Gleichgewichtswert abgesenkt wird, entscheidend. Die Dynamik des Tensidsystems ist dabei für die Benetzungsgeschwindigkeit von großer Bedeutung.

Aus dem Stand der Technik sind entsprechende Alkoxylate und Verfahren zu deren Herstellung bekannt.

EP 1 050 524 A offenbart ein Verfahren zur Herstellung von alkylendgruppenverschlossenen Alkyl- und/oder Alkenylpolyglykolethern, die erhalten werden durch Umsetzung von Alkoholen der Formel R¹O(EO)ₙ(PO)ₘH mit einem Dialkylcarbonat der Formel R³OCOOR⁴, wobei R¹ für einen linearen oder verzweigten, gesättigten oder ungesättigten Alkyl-, Alkenyl- und/oder Acylrest mit 6 bis 22 Kohlenstoffatomen und/oder gegebenenfalls mit einer Gruppe R² substituierten Kohlenwasserstoffrest, in der R² für Wasserstoff, C₁₋₁₂-Alkyl, Glyceryl oder den Rest eines Alkylglycols mit 2 bis 6 Kohlenstoffatomen, n für Zahlen von 0 bis 15 und m für Zahlen von 0 bis 10 steht, und R³ und R⁴ unabhängig voneinander für einen Alkylrest mit 1 bis 8 Kohlenstoffatomen steht.

In US 4,753,885 wir ein Alkoxylat der Formel R-O-(X¹)ₙ-(X²)ₘ-(X³)ₚ-Z, worin R ein Alkylrest mit 6 bis 22 Kohlenstoffatomen oder ein Alkylphenylrest, in dem die Alkyleinheit 6 bis 12 Kohlenstoffatome aufweist, X¹ und X³ Ethylenoxideinheiten n und p jeweils von 0 bis 15 und in Summe nicht kleiner als 2 sind, die Gruppen X² Propylenoxid und/oder Butylenoxideinheiten bedeuten, m 0 bis 15 und Z ein geradkettiger oder verzweigter Alkylrest mit 1 bis 14 Kohlenstoffatomen, Allyl oder Benzyl bedeuten kann, offenbart.

In US 4,973,423 werden Polyethylenglykolether der Formel R¹-O(EO)ₙ-R², worin R¹ ein linearer oder verzweigter C₆- bis C₁₈-Alkyl- oder Alkenylrest, R² ein C₄- bis C₈-Alkylrest und n eine ganze Zahl von 2 bis 6 bedeuten, offenbart. Des Weiteren wird die Verwendung dieser Verbindungen als entschäumende Additive für schaumarme Reinigungszusammensetzungen offenbart.

US 5,677,273 offenbart Benetzungsmittel-Zubereitungen zur Vorbehandlung von Textilien, welche gemischte Ether der Formel R¹O-(PO)ₘ(EO)ₙ-R², worin R¹ ein streng linearer Alkylrest mit 8 bis 10 Kohlenstoffatomen, R² ein Alkylrest mit 1 bis 4 Kohlenstoffatomen, m eine Zahl von 0,5 bis 2 und n eine Zahl von 6 bis 9 bedeuten.

In DE 36 26 567 A1 wird eine Aufzeichnungsflüssigkeit oder ein Aufzeichnungsverfahren offenbart, in dem ein Lösungsmittel der Formel R¹-(EO)ₘ-R², worin R¹ und R² jeweils unabhängig voneinander Alkylreste mit 1 bis 6 Kohlenstoffatomen oder Arylreste mit 8 oder weniger Kohlenstoffatomen bedeuten, und m eine ganze Zahl von 3 bis 20 ist, eingesetzt wird.

In US 3,407,142 A werden n-Butyl-ethyl-di-ether von Polypropylenglykolen offenbart, die in Kombination mit Tetraalkylorthosilikaten als Kühlflüssigkeit eingesetzt werden können.

In US 5,071,454 A wird ein Verfahren zur Entfernung polarer und unpolarer Lösungsmittel aus einem Gasstrom offenbart, indem der Gasstrom mit Lösungsmitteln enthaltend Di-n-butylether der Formel C₄H₉O-(CH₂CHRO)ₓ-C₄H₉ mit R = Wasserstoff oder Methyl und x eine Zahl von 1 bis 8, behandelt wird.

In T. P. Hobin "Model Polyethers 1-Synthesis by the Williamson Reaction, Polymer, Band 6, 1965, Seiten 403 bis 409 wird ein Verfahren zur Herstellung von Polyalkylenethern beschrieben. Die Polyalkylenether gemäß Tabelle 3 der genannten Schrift zeichnen sich dadurch aus, dass die Länge der Alkylen-Einheiten zwischen 2 Sauerstoffatomen der Länge der endständigen Alkylreste entspricht. So sind Verbindungen offenbart, die 3, 4, 5 und 6 Kohlenstoffatome in den Alkylen-Einheiten und in den endständigen Alkylresten aufweisen.

In Hiroatsu Matsuura et al., J. Mol. Structure, Band 189, 1988, Seiten 249 bis 256 werden Polyalkylenether offenbart, die Ethylenoxideinheiten und Butyl-, Hexyl- oder Octylreste als endständige Alkylreste aufweisen.

Y. Chen und G. L. Baker, Synthesis and Properties of ABA Amphiphilis, JOC, Band 64, Nummer 18, 1999, Seiten 6870 bis 6873 offenbart Polyethylenoxide, die jeweils Hexyl- oder Octylreste als endständige Alkylreste aufweisen.
- R: C₁₋₆-Alkyl oder Benzyl
- A: Ethylenoxy
- B: C₃₋₆-Alkylenoxy oder Gemische davon,
wobei Gruppen A und B statistisch verteilt, alternierend oder in Form zweier oder mehrerer Blöcke in beliebiger Reihenfolge vorliegen können,
- n: ganze Zahl im Bereich von 4 bis 8
- x: Zahl im Bereich von 3 bis 25
- y: Zahl im Bereich von 0 bis 10
wobei x + y mindestens 3 ist, durch Veretherung von Verbindungen der allgemeinen Formel (II)

CₙH₂ₙ₊₁O(A)ₓ(B)_{y}H (II)

mit den angegebenen Bedeutungen der Reste,
mit einem Alkylierungsmittel, das das Wasserstoffatom der endständigen Hydroxylgruppe durch R ersetzt, wobei sich die Verbindung der allgemeinen Formel (I) von Verbindungen der allgemeinen Formel (II)

CₙH₂ₙ₊₁O(A)ₓ(B)_{y}H (II)

in Form eines Alkylglykolalkoxylats oder -diglykolalkoxylats ableitet, das erhältlich ist durch Alkoxylierung von C₄₋₈-Alkylglykolen oder -diglykolen mit den Einheiten A und B zugrunde liegenden Alkylenoxiden.

Dabei ist R ein C₁₋₆-Alkylrest, der linear oder verzweigt sein kann, oder ein Benzylrest. Vorzugsweise ist R ein C₁₋₃-Alkylrest, insbesondere ein linearer C₁₋₃-Alkylrest, insbesondere Methyl, Ethyl oder Propyl, speziell Methyl.

B bedeutet C₃₋₆-Alkylenoxy oder Gemische davon, vorzugsweise Propylenoxy oder Butylenoxy, insbesondere Propylenoxy.

x ist eine Zahl im Bereich von 3 bis 25, bevorzugt 5 bis 15, insbesondere 5 bis 12.

y ist eine Zahl im Bereich von 0 bis 10, vorzugsweise 0, 1 oder 2.

Beim Rest CₙH₂ₙ₊₁ kann es sich um lineare oder einfach oder mehrfach verzweigte Alkylreste handeln, wobei auch Gemische von linearen oder verzweigten Alkylresten vorliegen können. Besonders bevorzugt liegt ein linearer und damit endständiger Alkylrest vor.

Die Werte von x und y stellen Mittelwerte dar, da bei der Alkoxylierung von Alkanolen in der Regel eine Verteilung des Alkoxylierungsgrades erhalten wird. Daher können x und y von ganzzahligen Werten abweichen. Die Verteilung des Alkoxylierungsgrades kann in gewissem Umfang durch Einsatz unterschiedlicher Alkoxylierungskatalysatoren eingestellt werden. Werden neben Ethylenoxid auch ein oder mehrere längerkettige Alkylenoxide zur Alkoxylierung eingesetzt, so können die unterschiedlichen Alkylenoxidreste statisch verteilt, alternierend oder in Form zweier oder mehrerer Blöcke in beliebiger Reihenfolge vorliegen. Besonders bevorzugt wird nur mit Ethylenoxid alkoxyliert, so dass ein reiner (Poly)ethylenoxid-Rest vorliegt. Der Mittelwert der homologen Verteilung wird durch die angegebenen Zahlen x und y dargestellt.

Die Alkoxylierung kann beispielsweise unter Verwendung von alkalischen Katalysatoren wie Alkalihydroxiden oder Alkalialkoholaten durchgeführt werden. Durch den Einsatz dieser Katalysatoren resultieren spezielle Eigenschaften, insbesondere der Verteilung des Alkoxylierungsgrades.

Die Alkoxylierung kann zudem unter Verwendung von Lewis-saurer Katalyse mit den daraus resultierenden speziellen Eigenschaften durchgeführt werden, insbesondere in Gegenwart von BF₃ x H₃PO₄, BF₃ Dietherat, BF₃, SbCl₅, SnCl₄ x 2 H₂O, Hydrotalcit. Geeignet als Katalysator sind auch Doppelmetallcyanid (DMC)-Verbindungen.

Dabei kann der überschüssige Alkohol abdestilliert werden, oder das Alkoxylat kann durch einen Zwei-Stufen-Prozess gewonnen werden. Auch die Herstellung gemischter Alkoxylate aus beispielsweise EO und PO ist möglich, wobei sich an den Alkanolrest zunächst ein Propylenoxid-Block und anschließend ein Ethylenoxid-Block anschließen können, oder zunächst ein Ethylenoxid-Block und sodann ein Propylenoxid-Block. Auch statistische/random-Verteilungen sind möglich. Bevorzugte Umsetzungsbedingungen sind nachstehend angegeben.

Vorzugsweise wird die Alkoxylierung durch starke Basen katalysiert, die zweckmäßigerweise in Form eines Alkalihydroxids oder Erdalkalihydroxids, in der Regel in einer Menge von 0,1 bis 1 Gew.-%. bezogen auf die Menge des Alkanols R2-OH, zugesetzt werden, (Vergl. G.Gee et al., J. Chem. Soc. (1961), S. 1345; B. Wojtech, Makromol. Chem. 66, (1966), S.180).

Auch eine saure Katalyse der Additionsreaktion ist möglich. Neben Bronstedsäuren eignen sich auch Lewissäuren, wie AlCl₃ oder BF₃. (Vergl. P.H.Plesch, The Chemistry of Cationic Polymerization, Pergamon Press, New York (1963)).

Als DMC-Verbindung können prinzipiell alle dem Fachmann bekannten geeigneten Verbindungen verwendet werden.

Als Katalysator geeignete DMC-Verbindungen sind beispielsweise in der WO 99/16775 und der DE-A-10117273 beschrieben. Insbesondere sind für die Alkoxylierung Doppelmetallcyanid-Verbindung der allgemeinen Formel als Katalysator geeignet:

M¹ₐ[M²(CN)_{b}(A)_{c}]_{d} · fM¹_{g}Xₙ · h(H₂O) · eL·kP,

in der
- M¹ mindestens ein Metallion, ausgewählt aus der Gruppe bestehend aus Zn²⁺, Fe²⁺, Fe³⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺ ist,
- M² mindestens ein Metallion, ausgewählt aus der Gruppe bestehend aus Fe²⁺, Fe³⁺, Co²⁺, Co³⁺, Mn²⁺, Mn³⁺, V⁴⁺, V⁵⁺, Cr²⁺, Cr³⁺, Rh³⁺, Ru²⁺, Ir³⁺ist,
- A und X unabhängig voneinander ein Anion, ausgewählt aus der Gruppe, bestehend aus Halogenid, Hydroxid, Sulfat, Carbonat, Cyanid, Thiocyanat, Isocyanat, Cyanat, Carboxylat, Oxalat, Nitrat, Nitrosyl, Hydrogensulfat, Phosphat, Dihydrogenphosphat, Hydrogenphosphat oder Hydrogencarbonat sind,
- L ein mit Wasser mischbarer Ligand ist, ausgewählt aus der Gruppe, bestehend aus Alkoholen, Aldehyden, Ketonen, Ethern, Polyethern, Estern, Polyestern, Polycarbonat, Harnstoffen, Amiden, primären, sekundären und tertiären Aminen, Liganden mit Pyridin-Stickstoff, Nitrilen, Sulfiden, Phosphiden, Phosphiten, Phosphanen, Phosphonaten und Phosphaten,
- k eine gebrochene oder ganze Zahl größer oder gleich Null ist, und
- P ein organischer Zusatzstoff ist,
- a, b, c, d, g und n so ausgewählt sind, dass die Elektroneutralität der Verbindung (I) gewährleistet ist, wobei c = 0 sein kann,
- e die Anzahl der Ligandenmoleküle eine gebrochenen oder ganze Zahl größer 0 oder 0 ist,
- f, h und m unabhängig voneinander eine gebrochene oder ganze Zahl größer 0 oder 0 sind.

Als organische Zusatzstoffe P sind zu nennen: Polyether, Polyester, Polycarbonate, Polyalkylenglykolsorbitanester, Polyakylenglykolglycidylether, Polyacrylamid, Poly(acrylamid-co-acrylsäure), Polyacrylsäure, Poly(acrytamid-co-maleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly(acrylsäure-co-styrol), Oxazolinpolymere, Polyalkylenimine, Maleinsäure- und Maleinsäureanhydridcopolymere, Hydroxyethylcellulose, Polyacetate, ionische oberflächen- und grenzflächenaktive Verbindungen, Gallensäure oder deren Salze, Ester oder Amide, Carbonsäureester mehrwertiger Alkohole und Glycoside.

Diese Katalysatoren können kristallin oder amorph sein. Für den Fall, dass k gleich null ist, sind kristalline Doppelmetallcyanid-Verbindungen bevorzugt. Im Fall, dass k größer null ist, sind sowohl kristalline, teilkristalline, als auch substantiell amorphe Katalysatoren bevorzugt.

Von den modifizierten Katalysatoren gibt es verschiedene bevorzugte Ausführungsformen. Eine bevorzugte Ausführungsform sind Katalysatoren der Formel, bei denen k größer null ist. Der bevorzugte Katalysator enthält dann mindestens eine Doppelmetallcyanid-Verbindung, mindestens einen organischen Liganden und mindestens einen organischen Zusatzstoff P.

Bei einer anderen bevorzugten Ausführungsform ist k gleich null, optional ist e auch gleich null und X ist ausschließlich ein Carboxylat, bevorzugt Formiat, Acetat und Propionat. Derartige Katalysatoren sind in der WO 99/16775 beschrieben. Bei dieser Ausführungsform sind kristalline Doppelmetallcyanid-Katalysatoren bevorzugt. Ferner bevorzugt sind Doppelmetallcyanid-Katalysatoren, wie in der WO 00/74845 beschrieben, die kristallin und plättchenförmig sind.

Die Herstellung der modifizierten Katalysatoren erfolgt durch Vereinigung einer Metallsalz-Lösung mit einer Cyanometallat-Lösung, die optional sowohl einen organischen Liganden L als auch einen organischen Zusatzstoff P enthalten können. Anschließend werden der organische Ligand und optional der organische Zusatzstoff zugegeben. Bei einer bevorzugten Ausführungsform der Katalysatorherstellung wird zunächst eine inaktive Doppelmetallcyanid-Phase hergestellt und diese anschließend durch Umkristallisation in eine aktive Doppelmetallcyanidphase überführt, wie in der PCT/EP01/01893 beschrieben.

Bei einer anderen bevorzugten Ausführungsform der Katalysatoren sind f, e und k ungleich Null. Dabei handelt es sich um Doppelmetallcyanid-Katalysatoren, die einen mit Wasser mischbaren organischen Ligand (im allgemeinen in Mengen von 0,5 bis 30 Gew.%) und einen organischen Zusatzstoff (im allgemeinen in Mengen von 5 bis 80 Gew.%) enthalten wie in der WO 98/06312 beschrieben. Die Katalysatoren können entweder unter starkem Rühren (24000U/Min mit Turrax) oder unter Rühren hergestellt werden wie in der US 5,158,922 beschrieben.

Insbesondere als Katalysator geeignet sind für die Alkoxylierung Doppelmetallcyanid-Verbindungen, die Zink, Kobalt oder Eisen oder zwei davon enthalten. Besonders geeignet ist beispielsweise Berliner Blau.

Bevorzugt werden kristalline DMC-Verbindungen eingesetzt. In einer bevorzugten Ausführungsform wird eine kristalline DMC-Verbindung vom Zn-Co-Typ als Katalysator verwendet, der als weitere Metallsalzkomponente Zinkacetat enthält. Derartige Verbindungen kristallisieren in monokliner Struktur und weisen einen plättchenförmigen Habitus auf. Derartige Verbindungen werden beispielsweise in der WO 00/74845 oder der PCT/EP01/01893 beschrieben.

Als Katalysator geeignete DMC-Verbindungen können prinzipiell auf alle dem Fachmann bekannten Arten hergestellt werden. Beispielsweise können die DMC-Verbindungen durch direkte Fällung, "incipient wetness"-Methode, durch Herstellung einer Precursor-Phase und anschließende Umkristallisation hergestellt werden.

Die DMC-Verbindungen können als Pulver, Paste oder Suspension eingesetzt werden oder zu einem Formkörper verformt werden, in Formkörpern, Schäume oder ähnliches eingebracht werden oder auf Formkörper, Schäume oder ähnliches aufgebracht werden.

Die zur Alkoxylierung eingesetzte Katalysator-Konzentration bezogen auf das Endmengengerüst ist typischerweise kleiner als 2000 ppm, bevorzugt kleiner als 1000 ppm, insbesondere kleiner als 500 ppm, besonders bevorzugt kleiner als 100 ppm, beispielsweise kleiner als 50 ppm.

Die Additionsreaktion wird bei Temperaturen von etwa 90 bis etwa 240°C, vorzugsweise von 120 bis 180°C, im geschlossenen Gefäß ausgeführt. Das Alkylenoxid oder die Mischung verschiedener Alkylenoxide wird der Mischung aus erfindungsgemäßem Alkanolgemisch und Alkali unter dem bei der gewählten Reaktionstemperatur herrschenden Dampfdruck des Alkylenoxidgemisches zugeführt. Gewünschtenfalls kann das Alkylenoxid mit bis zu etwa 30 bis 60 % mit einem Inertgas verdünnt werden. Dadurch wird eine zusätzliche Sicherheit gegen explosionsartige Polyaddition des Alkylenoxids gegeben.

Wird ein Alkylenoxidgemisch eingesetzt, so werden Polyetherketten gebildet in denen die verschiedenen Alkylenoxidbausteine praktisch statistisch verteilt sind. Variationen in der Verteilung der Bausteine längs der Polyetherkette ergeben sich aufgrund unterschiedlicher Reaktionsgeschwindigkeiten der Komponenten und können auch willkürlich durch kontinuierliche Zufuhr einer Alkylenoxidmischung programmgesteuerter Zusammensetzung erreicht werden. Werden die verschiedenen Alkylenoxide nacheinander zur Reaktion gebracht so erhält man Polyetherketten, mit blockartiger Verteilung der Alkylenoxid-Bausteine.

Die Länge der Polyetherketten schwankt innerhalb des Reaktionsprodukts statistisch um einen Mittelwert, der im Wesentlichen dem sich aus der Zusatzmenge ergebenden stöchiometrischen Wert entspricht.

Zur Herstellung der Verbindungen der allgemeinen Formel (I) werden Alkylglykolalkoxylate oder -diglykolakoxylate eingesetzt, die erhältlich sind durch Alkoxylierung von C₄₋₈-Alkylglykolen oder -diglykolen mit C₂₋₅-Alkoxiden, vorzugsweise bis zu einem mittleren Alkoxylierungsgrad von 1 bis 24 bzw. 23, bezogen auf die C₄₋₈-Alkylglykole oder - diglykole. Diese werden sodann verethert, wobei in der endständigen Hydroxylgruppe H durch R ersetzt wird.

Die nachstehenden Ausführungen beziehen sich ebenso auf Alkyldiglykole wie auf Alkylglykole bzw. deren Alkoxylate.

Bei den zugrundeliegenden Alkylglykolen kann es sich um lineare oder verzweigte Alkylglykole handeln. Die Anbindung des C₄₋₈-Alkylrestes an den Glykol kann endständig oder an einer anderen Position entlang der Alkylkette erfolgen. Vorzugsweise handelt es sich um lineare Alkylglykole, insbesondere um lineare, endständige Alkylglykole. Vorzugsweise weisen die Alkylreste der Alkylglykole 5 bis 8 Kohlenstoffatome auf. Der Alkoxylierungsgrad beträgt vorzugsweise im Mittel 2 bis 24 bzw. 1 bis 23, besonders bevorzugt 4 bis 14 bzw. 3 bis 13, bezogen auf die Glykole bzw. Diglykole. Zur Alkoxylierung können dabei vorzugsweise C₂₋₄-Alkoxide eingesetzt werden. Vorzugsweise werden Ethylenoxid, Propylenoxid, Butylenoxid oder Gemische davon eingesetzt. Besonders bevorzugt wird Ethylenoxid eingesetzt. Die bevorzugten Bereiche beziehen sich auch auf die Alkylglykolalkoxylate und Alkyldiglykolalkoxylate an sich.

Die Herstellung erfolgt hier ausgehend von alkoholfreien, vorzugsweise reinen Alkylglykolen und Alkyldiglykolen und nicht, wie vorstehend beschrieben, ausgehend von Alkanolen, durch Alkoxylierung. Daher enthalten die Produktgemische auch keine verbleibenden Alkanole, sondern höchstens Alkylglykole. Es ergibt sich eine für Alkylglykole spezifische Verteilung des Alkoxylierungsgrades. Durch das Herstellungsverfahren sind die Alkylglykolalkoxylate frei von Alkoholen.

Alkoxylate sind oligomere oder polymere Umsetzungsprodukte mit Alkoxiden. Aufgrund der dem Fachmann bekannten Kinetik von Polymerisationen kommt es zwangsläufig zu einer statistischen Verteilung von Homologen, deren Mittelwert üblicherweise angegeben wird. Die Häufigkeitsverteilung der Homologen beinhaltet insbesondere bei niedrigen Alkoxylierungsgraden den Ausgangsstoff. Durch die Wahl des Katalysators kann zwar die Verteilung in gewissem Umfang beeinflusst werden, am Prinzip der Verteilungskurve ändert sich aber nichts. Reine Alkyloligoglykole lassen sich nur durch destillative oder chromatographische Aufarbeitung herstellen und sind daher teuer. Außerdem hat sich gezeigt, dass die Verteilung der Homologen einen vorteilhaften Einfluss auf das Aggregationsverhalten hat.

Die in dieser Ausführungsform beschriebenen veretherten Alkoxylate besitzen die für das Aggregationsverhalten und die anderen erfindungsgemäßen Eigenschaften wichtige Homologenverteilung, ohne Alkohol zu enthalten.

Die nach den anderen Verfahren erhaltenen Produkte werden von verbliebenen Alkanolen befreit.

Der Ausdruck "alkanolfrei" bezieht sich auf Alkoxylate, die keine durch Gaschromatographie (GC) messbaren Mengen an Alkanolen, insbesondere CₙH₂ₙ₊₁OH, aufweisen.

Die Bestimmung der Verteilung der Alkoxylierungsgrade kann durch chromatographische Verfahren erfolgen.

Für einen Vergleich zwischen Alkanolalkoxylaten und Alkylglykolalkoxylaten wird auf WO 03/60049 verwiesen.

Da im Produktgemisch keine Alkohole vorliegen, ist es weitgehend geruchsfrei. Die Verbindungen der Formel (I) können - insbesondere in den genannten Anwendungen - in Kombination mit Tensiden eingesetzt werden. Als Tenside können erfindungsgemäß alle Tenside eingesetzt werden, die in einer Menge von 5 g/l Wasser gelöst eine Grenzflächenspannung von weniger als 45 mN/m bei 20°C zeigen. Bei den Tensiden kann es sich allgemein um alkoxylierte Alkohole, Amide, Säuren, Betaine, Aminoxide oder Amine, aber auch um Dihydroxyalkine und Derivate und Gemische davon handeln. Die Geschwindigkeit der Einstellung des Endniveaus der Grenzflächenspannung kann dabei von der Molekülarchitektur, wie der Kettenlänge und dem Verzweigungsgrad des Alkohols, der Länge und Solvatisierung des Alkoxylats, der Tensidkonzentration und der Tensidaggregation abhängen. Im allgemeinen diffundieren kleinere Aggregate schneller als große Aggregate.

Vorzugsweise sind die Tenside nicht-ionische Tenside und ausgewählt aus C₂₋₅-, vorzugsweise C₂₋₄-Alkoxylaten von C₉₋₂₀-, vorzugsweise C₉₋₁₅-, insbesondere C₉₋₁₃-Alkanolen, die im Mittel einen Alkoxylierungsgrad von 3 bis 40, vorzugsweise 4-15, insbesondere 5 bis 12 aufweisen, und Mischungen davon. Dabei kann es sich um lineare oder verzweigte Alkanole handeln. Bei einem verzweigten Alkohol liegt der Verzweigungsgrad vorzugsweise im Bereich von 1,1 bis 1,5. Die Alkoxylierung kann mit beliebigen C₂₋₄-Alkoxiden und Gemischen davon erfolgen. Beispielsweise kann mit Ethylenoxid, Propylenoxid oder Butylenoxid alkoxyliert werden. Besonders bevorzugt werden Ethylenoxid, Propylenoxid oder Gemische davon eingesetzt. Insbesondere bevorzugt ist Ethylenoxid. Derartige nicht-ionische Tenside sind bekannt und beispielsweise in EP-A 0 616 026 und EP-A 0 616 028 beschrieben. In diesen Schriften sind auch kürzerkettige Alkylalkoxylate erwähnt.

Die als Tenside eingesetzten nicht-ionischen Tenside können auch durch Dihydroxyalkine oder Derivate davon ersetzt sein. Es kann sich ferner um schaumarme oder schaumdämpfende Tenside handeln, vgl. auch EP-A 0 681 865. Schaumarme und schaumdämpfende Tenside sind dem Fachmann bekannt.

Die Erfindung betrifft auch die Verwendung der erfindungsgemäß hergestellten alkanolfreien Alkoxylate in alkalischen, Löse- oder Verdünnungsmittel enthaltenden Formulierungen zur Verbesserung der Netzwirkung der Formulierungen eingesetzt. Die Formulierungen enthalten in der Regel Wasser oder organische Löse- oder Verdünnungsmittel. Der pH-Wert dieser Formulierungen beträgt vorzugsweise mehr als 10, bevorzugt mehr als 12.

Geeignete Formulierungen enthalten beispielsweise die Alkoxylate sowie mindestens ein Tensid und Wasser.

Die Erfindung betrifft ferner ein Entfettungs-, Metallreinigungs- oder Netzmittel oder Pflanzenschutzformutierung, enthaltend ein erfindungsgemäß hergestelltes Alkoxylat.

Die Erfindung betrifft ferner Lacke, Formulierungen für Sprühanwendungen, die Mineralaufbereitung, die Papier- und Holzindustrie oder für Überzüge, Beschichtungs-, Klebe-, Lederbehandlungs-, Metallbehandlungs, Metallbearbeitungs- oder Textilbehandlungsmittel, enthaltend ein erfindungsgemäß hergestelltes Alkoxylat.

Bei dem Beschichtungsmittel kann es sich beispielsweise um eine Formulierung für Sprühanwendungen handeln.

Bei einer Lackformulierung kann es sich insbesondere um eine Lackformulierung handeln, die durch blade coating, spray coating oder curtain coating aufgebracht wird.

Die Aufgabe wird ferner erfindungsgemäß gelöst durch die Verwendung der erfindungsgemäß hergestellten Alkoxylate zur Verminderung der Grenzflächenspannung, insbesondere bei kurzen Zeiten von üblicherweise unter 1 Sekunde, und Beschleunigung der Einstellung der Grenzflächenspannung in wässrigen Tensidformulierungen oder wässrigen Dispersionen.

Die Aufgabe wird ferner gelöst durch die Verwendung der erfindungsgemäß hergestellten Alkoxylate zur Erhöhung der Benetzungsgeschwindigkeit in wässrigen Netzmitteln.

Die erfindungsgemäß hergestellten Alkoxylate können zur Formulierung von Mitteln in allen Bereichen dienen, in denen hochdynamische Formulierungen, insbesondere unter alkalischen Bedingungen, verwendet werden. Beispiele dafür sind:
- Allzweckreiniger, Sprühreiniger zur Reinigung im industriellen und institutionellen Bereich einschließlich der Metallbearbeitung,
- Druckwalzen- und Druckplattenreinigungsmittel in der Druckindustrie,
- Lacke, Farbformulierungen, Beschichtungsmittel, z.B. für Papier wie Papierstreichfarben, Holzbindemittel, besonders Formaldehydharze, Klebstoffe in der Lack- und Folienindustrie, z.B. in Dispersionsform,
- Formulierungen für Sprühanwendungen,
- Metallbehandlung beziehungsweise -bearbeitung wie Korrosionsschutzformulierungen, Schneid-, Schleif- oder Bohrhilfsmittel, Bohrflüssigkeiten und Schmiermittel beziehungsweise Kühlschmiermittel,
- Formulierungen in der Textilindustrie,
- Formulierungen zum Auslaugen von Erzen z.B. nach dem Cyanidverfahren,
- Formulierungen zur Hydrophilierung von Pigmenten oder Partikeln zur Bindung von feinteiligen Pigmenten und Stärken.

Solche Formulierungen enthalten üblicherweise weitere Inhaltsstoffe wie Tenside, Komplexbildner, Polymere und andere Inhaltsstoffe.

Allgemein können die erfindungsgemäßen Mischungen in allen Bereichen eingesetzt werden, in denen die Wirkung von grenzflächenaktiven Stoffen, speziell unter alkalischen Bedingungen, notwendig ist.

Durch die Verwendung nicht-tensidischer Strukturen weisen die erfindungsgemäßen Formulierungen eine bessere Umwelt- und Hautverträglichkeit auf im Vergleich zu beispielsweise in EP-A 0 616 026 beschriebenen Systemen. Im Unterschied zu den gängigen Solubilisatoren wie Cumolsulfonat erfolgt die Wechselwirkung spezifisch mit den Tensiden. Damit greifen die erfindungsgemäß eingesetzten alkoxylierten Alkylglykole aktiv in die Belegung der Grenzfläche ein und beschleunigen die Einstellung des Grenzflächengleichgewichts.

Es ist erfindungsgemäß nicht notwendig und nicht erwünscht, dass ein Restgehalt an Alkohol in den erfindungsgemäßen Mischungen oder Formulierungen vorliegt. Gemäß einer Ausführungsform sind die erfindungsgemäßen Mischungen, Mittel und Formulierungen frei von Alkoholen und bevorzugt auch von Alkylglykolen bzw. Diglykolen, insbesondere von C₄₋₈-Alkylglykolen und C₉₋₁₃-Alkanolen. Erfindungsgemäß wurde gefunden, dass ohne einen Restgehalt an Alkohol, der üblicherweise bei niedrigen Alkoholalkoxylaten herstellungsbedingt im Produkt enthalten ist, durch Einsatz der erfindungsgemäßen Alkylglykolalkoxylate Tensidformulierungen mit hoher Grenzflächendynamik formuliert werden können.

Die erfindungsgemäße Netzwirkung kann dabei durch eine dynamische Messung der Oberflächenspannung, beispielsweise durch Verwendung eines Blasendrucktensiometers, bestimmt werden. Ein entsprechendes Vorgehen ist beispielsweise in S.S. Dukhen, G. Kretzschmar, R. Miller (Ed.), Dynamics of adsorption at liquid interfaces, Elsevier, 1995, beschrieben. Die Netzwirkung auf Oberflächen kann dabei durch eine dynamische Messung der Grenzflächenspannung bestimmt werden. Eine derartige Methode ist die videogestützte, zeitaufgelöste Randwinkelmessung.

Die Erfindung betrifft auch die Verwendung der erfindungsgemäß hergestellten Alkoxylate in der Oberflächenveredelung. Die Erfindung betrifft damit ein Beschichtungsmittel, bei dem es sich um eine wässrige Papierstrich-Dispersion handelt, die Wasser, Pigmente, Bindemittel und 0,05 bis 5 Gew.-%, bezogen auf die Pigmente, an erfindungsgemäß hergestellten Alkoxylaten enthält. Die Formulierungen können dabei natürliche oder synthetische Bindemittel oder deren Gemische enthalten. Weitere mögliche Inhaltsstoffe sind Rheologiehilfsmittel, Dispergiermittel, Verdicker etc.

In den Beschichtungsmitteln kann nun die Tröpfchengröße im "spray coating"-Prozeß signifikant beeinflusst werden, bei gleichzeitig niedriger Schaumarmut der Sprüh-Lacke oder -Farbformulierungen.

Die in den Beschichtungsmitteln eingesetzten Pigmente stellen üblicherweise die Hauptkomponente dar. Es können alle üblicherweise eingesetzten Pigmente wie Calciumcarbonate, Kaolin, Talk, Titandioxid, Gips, Kreide, Ruß oder synthetische Pigmente allein oder in Mischung verwendet werden.

Das Alkoxylat wird üblicherweise in Mengen von 0,05 bis 5%, bezogen auf die Formulierung, eingesetzt, bevorzugt in Mengen von 0,1 bis 2%. Dabei kann das Alkoxylat sowohl während des Herstellprozesses der Formulierung direkt (und/oder) als auch in einer Mischung mit einem Bestandteil der Streichfarbe (z.B. einer Pigmentslurry und/oder einem Bindemittel) zugegeben werden.

Die Erfindung wird nachstehend anhand von Beispielen näher erläutert:

### Referenzbeispiel 1

### Chemische Stabilität

Ein Alkylalkoxylat (Hexanol + 9 EO, mit Dimethylsulfat methyliert) gemäß Stand der Technik wird in 5%iger NaOH gelöst. Eine GPC-Untersuchung nach verschiedenen Zeiten ergibt, dass keine Veränderung der Peakstruktur beobachtet werden kann.

### Referenzbeispiel 2

### Grenzflächenaktive Wirkung

Von einer 0,5%igen Lösung eines Alkoxylats (s.o.) des Standes der Technik wird die Grenzflächenspannung zeitaufgelöst mit dem Blasendrucktensiometer (Tz. H. lliev, C.D. Dushkin, Colloid Polym. Sci 270 (1992) 370) bestimmt. Man findet eine Erniedrigung der Grenzflächenspannung von 72 mN/m auf 32 mN/m innerhalb der ersten 20 ms.

## Patentansprüche

1. Verfahren zur Herstellung von alkanoffreien Alkoxylaten der allgemeinen Formel (I)
CₙH₂ₙ₊₁O(A)ₓ(B)_{y}R (I)
mit der Bedeutung
R C₁₋₆-Alkyl oder Benzyl
A Ethylenoxy
B C₃₋₆-Alkylenoxy oder Gemische davon,
wobei Gruppen A und B statistisch verteilt, alternierend oder in Form zweier oder mehrerer Blöcke in beliebiger Reihenfolge vorliegen können,
n ganze Zahl im Bereich 4 bis 8
x Zahl im Bereich von 3 bis 25
y Zahl im bereich von 0 bis 10,
wobei x+y mindestens 3 ist, durch Veretherung von Verbindungen der allgemeinen Formel (II)
CₙH₂ₙ₊₁O(A)ₓ(B)_{y}H (II)
mit den angegebenen Bedeutungen der Reste,
mit einem Alkylierungsmittel, das das Wasserstoffatom der endständigen Hydroxylgruppe durch R ersetzt, wobei sich die Verbindung der allgemeinen Formel (I) von Verbindungen der allgemeinen Formel (II)
CₙH₂ₙ₊₁O(A)ₓ(B)_{y}H (II)
in Form eines Alkylglykolalkoxylats oder -diglykolalkoxylats ableitet, das erhältlich ist durch Alkoxylierung von C₄₋₈-Alkylglykolen oder -diglykolen mit den Einheiten A und B zugrunde liegenden Alkylenoxiden.

2. Verwendung von alkanolfreien Alkoxylaten der allgemeinen Formel (I)
CₙH₂ₙ₊₁O(A)ₓ(B)_{y}R (I)
mit der Bedeutung
R C₁₋₆-Alkyl oder Benzyl
A Ethylenoxy
B C₃₋₆-Alkylenoxy oder Gemische davon,
wobei Gruppen A und B statistisch verteilt, alternierend oder in Form zweier oder mehrerer Blöcke in beliebiger Reihenfolge vorliegen können,
n ganze Zahl im Bereich 4 bis 8
x Zahl im Bereich von 3 bis 25
y Zahl im bereich von 0 bis 10,
wobei x+y mindestens 3 ist, hergestellt nach dem Verfahren gemäß Anspruch 1 in alkalischen, Löse- oder Verdünnungsmittel, bevorzugt Wasser, enthaltenden Formulierungen zur Verbesserung der Netzwirkung der Formulierungen.

3. Verwendung von alkanolfreien Alkoxylaten der allgemeinen Formel (I)
CₙH₂ₙ₊₁O(A)ₓ(B)_{y}R (I)
mit der Bedeutung
R C₁₋₆-Alkyl oder Benzyl
A Ethylenoxy
B C₃₋₆-Alkylenoxy oder Gemische davon,
wobei Gruppen A und B statistisch verteilt, alternierend oder in Form zweier oder mehrerer Blöcke in beliebiger Reihenfolge vorliegen können,
n ganze Zahl im Bereich 4 bis 8
x Zahl im Bereich von 3 bis 25
y Zahl im bereich von 0 bis 10,
wobei x+y mindestens 3 ist, hergestellt nach dem Verfahren gemäß Anspruch 1 zur Verminderung der Grenzflächenspannung und Beschleunigung der Einstellung der Grenzflächenspannung in wässrigen Tensidformulierungen oder wässrigen Dispersionen, zur Hydrophilierung von Pigmenten oder Partikeln oder zur Bindung von feinteiligen Pigmenten und Stäuben.

4. Entfettungs-, Metallreinigungs- oder Netzmittel oder Pflanzenschutzformulierung, enthaltend ein alkanolfreies Alkoxylat der allgemeinen Formel (I)
CₙH₂ₙ₊₁O(A)ₓ(B)_{y}R (I)
mit der Bedeutung
R C₁₋₆-Alkyl oder Benzyl
A Ethylenoxy
B C₃₋₆-Alkylenoxy oder Gemische davon,
wobei Gruppen A und B statistisch verteilt, alternierend oder in Form zweier oder mehrerer Blöcke in beliebiger Reihenfolge vorliegen können,
n ganze Zahl im Bereich 4 bis 8
x Zahl im Bereich von 3 bis 25
y Zahl im bereich von 0 bis 10,
wobei x+y mindestens 3 ist, hergestellt nach dem Verfahren gemäß Anspruch 1.

5. Lacke, Formulierungen für Sprühanwendungen, die Mineralaufbereitung, die Papier- und Holzindustrie oder für Überzüge, Beschichtungs-, Klebe-, Lederbehandlungs-, Metallbehandlungs, Metallbearbeitungs- oder Textilbehandlungsmittel, enthaltend ein alkanolfreies Alkoxylat der allgemeinen Formel (I)
CₙH₂ₙ₊₁O(A)ₓ(B)_{y}R (I)
mit der Bedeutung
R C₁₋₆-Alkyl oder Benzyl
A Ethylenoxy
B C₃₋₆-Alkylenoxy oder Gemische davon,
wobei Gruppen A und B statistisch verteilt, alternierend oder in Form zweier oder mehrerer Blöcke in beliebiger Reihenfolge vorliegen können,
n ganze Zahl im Bereich 4 bis 8
x Zahl im Bereich von 3 bis 25
y Zahl im bereich von 0 bis 10,
wobei x+y mindestens 3 ist, hergestellt nach dem Verfahren gemäß Anspruch 1.

6. Verwendung von alkanolfreien Alkoxylaten der allgemeinen Formel (I)
CₙH₂ₙ₊₁O(A)ₓ(B)_{y}R (I)
mit der Bedeutung
R C₁₋₆-Alkyl oder Benzyl
A Ethylenoxy
B C₃₋₆-Alkylenoxy oder Gemische davon,
wobei Gruppen A und B statistisch verteilt, alternierend oder in Form zweier oder mehrerer Blöcke in beliebiger Reihenfolge vorliegen können,
n ganze Zahl im Bereich 4 bis 8
x Zahl im Bereich von 3 bis 25
y Zahl im bereich von 0 bis 10,
wobei x+y mindestens 3 ist, hergestellt nach dem Verfahren gemäß Anspruch 1 zur Erhöhung der Benetzungsgeschwindigkeit in wässrigen Netzmitteln.

7. Beschichtungsmittel nach Anspruch 5, **dadurch gekennzeichnet, dass** es sich um eine wässrige Formulierung handelt, die Wasser, Pigmente, Bindemittel und 0,05 bis 5 Gew.-%, bezogen auf die Pigmente, eines alkanolfreien Alkoxylat der allgemeinen Formel (I)
CₙH₂ₙ₊₁O(A)ₓ(B)_{y}R (I)
mit der Bedeutung
R C₁₋₆-Alkyl oder Benzyl
A Ethylenoxy
B C₃₋₆-Alkylenoxy oder Gemische davon,
wobei Gruppen A und B statistisch verteilt, alternierend oder in Form zweier oder mehrerer Blöcke in beliebiger Reihenfolge vorliegen können,
n ganze Zahl im Bereich 4 bis 8
x Zahl im Bereich von 3 bis 25
y Zahl im bereich von 0 bis 10,
wobei x+y mindestens 3 ist, hergestellt nach dem Verfahren gemäß Anspruch 1, enthält.

8. Beschichtungsmittel nach Anspruch 7, **dadurch gekennzeichnet, dass** es sich um eine Formulierung für Sprühanwendungen handelt.

9. Lackformulierung nach Anspruch 5, **dadurch gekennzeichnet, dass** es sich um eine Lackformulierung handelt, die durch blade coating oder curtain coating aufgebracht wird.

## Claims

1. A process for preparing an alkanol-free alkoxylate of the general formula (I)
CₙH₂ₙ₊₁O(A)ₓ(B)_{y}R (I)
where
R is C₁₋₆ alkyl or benzyl
A is ethyleneoxy
B is C₃₋₆ alkyleneoxy or mixtures thereof,
it being possible for groups A and B to be in random distribution, in alternation or in the form of two or more blocks in any order,
n is an integer in the range from 4 to 8
x is a number in the range from 3 to 25
y is a number in the range from 0 to 10
and x + y is at least 3 by etherifying a compound of the general formula (II)
CₙH₂ₙ₊₁O(A)ₓ(B)_{y}H (II)
with the stated definitions of the radicals
with an alkylating agent which replaces the hydrogen atom of the terminal hydroxyl group by R, wherein the compound of the general formula (I) derives from compounds of the general formula (II)
CₙH₂ₙ₊₁O(A)ₓ(B)_{y}H (II)
in the form of an alkyl glycol alkoxylate or alkyl diglycol alkoxylate obtainable by alkoxylating C₄₋₈ alkyl glycols or diglycols with parent alkylene oxides of the units A and/or B.

2. The use of an alkanol-free alkoxylate of the general formula (I)
CₙH₂ₙ₊₁O(A)ₓ(B)_{y}R (I)
where
R is C₁₋₆ alkyl or benzyl
A is ethyleneoxy
B is C₃₋₆ alkyleneoxy or mixtures thereof,
it being possible for groups A and B to be in random distribution, in alternation or in the form of two or more blocks in any order,
n is an integer in the range from 4 to 8
x is a number in the range from 3 to 25
y is a number in the range from 0 to 10
and x + y is at least 3, prepared according to the process of claim 1 in an alkaline formulation comprising solvent or diluent, preferably water to improve the wetting action of the formulation.

3. The use of an alkanol-free alkoxylate of the general formula (I)
CₙH₂ₙ₊₁O(A)ₓ(B)_{y}R (I)
where
R is C₁₋₆ alkyl or benzyl
A is ethyleneoxy
B is C₃₋₆ alkyleneoxy or mixtures thereof,
it being possible for groups A and B to be in random distribution, in alternation or in the form of two or more blocks in any order,
n is an integer in the range from 4 to 8
x is a number in the range from 3 to 25
y is a number in the range from 0 to 10
and x + y is at least 3, prepared according to the process of claim 1 for reducing the surface tension and accelerating the establishment of the surface tension in aqueous surfactant formulations or aqueous dispersions, for hydrophilicizing pigments or particles or for binding finely divided pigments and dusts.

4. A degreasing, metal-cleaning or wetting composition or crop protection formulation comprising an alkanol-free alkoxylate of the general formula (I)
CₙH₂ₙ₊₁O(A)ₓ(B)_{y}R (I)
where
R is C₁₋₆ alkyl or benzyl
A is ethyleneoxy
B is C₃₋₆ alkyleneoxy or mixtures thereof,
it being possible for groups A and B to be in random distribution, in alternation or in the form of two or more blocks in any order,
n is an integer in the range from 4 to 8
x is a number in the range from 3 to 25
y is a number in the range from 0 to 10
and x + y is at least 3, prepared according to the process of claim 1.

5. A varnish, formulation for spray applications, mineral processing, the paper and wood industry or for covering, coating composition, adhesive composition, leather treatment composition, metal treatment composition, metalworking composition or textile treatment composition comprising an alkanol-free alkoxylate of the general formula (I)
CₙH₂ₙ₊₁O(A)ₓ(B)_{y}R (I)
where
R is C₁₋₆ alkyl or benzyl
A is ethyleneoxy
B is C₃₋₆ alkyleneoxy or mixtures thereof,
it being possible for groups A and B to be in random distribution, in alternation or in the form of two or more blocks in any order,
n is an integer in the range from 4 to 8
x is a number in the range from 3 to 25
y is a number in the range from 0 to 10
and x + y is at least 3, prepared according to the process of claim 1.

6. The use of an alkanol-free alkoxylate of the general formula (I)
CₙH₂ₙ₊₁O(A)ₓ(B)_{y}R (I)
where
R is C₁₋₆ alkyl or benzyl
A is ethyleneoxy
B is C₃₋₆ alkyleneoxy or mixtures thereof,
it being possible for groups A and B to be in random distribution, in alternation or in the form of two or more blocks in any order,
n is an integer in the range from 4 to 8
x is a number in the range from 3 to 25
y is a number in the range from 0 to 10
and x + y is at least 3, prepared according to the process of claim 1 for increasing the wetting rate in aqueous wetting compositions.

7. The coating composition according to claim 5, which is an aqueous formulation comprising water, pigments, binder and from 0.05 to 5% by weight, based on the pigments, of an alkanol-free alkoxylate of the general formula (I)
CₙH₂ₙ₊₁O(A)ₓ(B)_{y}R (I)
where
R is C₁₋₆ alkyl or benzyl
A is ethyleneoxy
B is C₃₋₆ alkyleneoxy or mixtures thereof,
it being possible for groups A and B to be in random distribution, in alternation or in the form of two or more blocks in any order,
n is an integer in the range from 4 to 8
x is a number in the range from 3 to 25
y is a number in the range from 0 to 10
and x + y is at least 3, prepared according to the process of claim 1.

8. The coating composition according to claim 7, which is a formulation for spray applications.

9. The coating formulation according to claim 5, which is a coating formulation applied by blade coating or curtain coating.

## Revendications

1. Procédé pour la préparation d'alcoolates exempts d'alcanols, de formule générale (I)
CₙH₂ₙ₊₁O(A)ₓ(B)_{y}R (I)
dans laquelle
R représente un groupe alkyle en C₁-C₆ ou benzyle
A représente un groupe éthylène-oxy
B représente un groupe alkylène(C₃-C₆)oxy ou des mélanges de tels groupes,
les groupes A et B pouvant être présents en distribution statistique, en alternance ou sous forme de deux ou plus de deux séquences en un ordre quelconque,
n représente un nombre entier allant de 4 à 8
x représente un nombre allant de 3 à 25
y représente un nombre allant de 0 à 10,
la somme x+y étant au moins égale à 3,
par éthérification de composés de formule générale (II)
CₙH₂ₙ₊₁O(A)ₓ(B)_{y}H (II)
où les radicaux ont les significations indiquées,
avec un agent d'alkylation qui remplace par R l'atome d'hydrogène du groupe hydroxy en bout de chaîne, le composé de formule générale (I) dérivant de composés de formule générale (II)
CₙH₂ₙ₊₁O(A)ₓ(B)_{y}H (II)
sous forme d'un alcoolate d'alkylglycol ou d'alkyldiglycol qui peut être obtenu par alcoxylation d'alkyl(C₄-C₈)glycols ou -diglycols avec les oxydes d'alkylène qui sont à la base des groupes A et B.

2. Utilisation d'alcoolates exempts d'alcanols, de formule générale (I)
CₙH₂ₙ₊₁O(A)ₓ(B)_{y}R (I)
dans laquelle
R représente un groupe alkyle en C₁-C₆ ou benzyle
A représente un groupe éthylène-oxy
B représente un groupe alkylène(C₃-C₆)oxy ou des mélanges de tels groupes,
les groupes A et B pouvant être présents en distribution statistique, en alternance ou sous forme de deux ou plus de deux séquences en un ordre quelconque,
n représente un nombre entier allant de 4 à 8
x représente un nombre allant de 3 à 25
y représente un nombre allant de 0 à 10,
la somme x+y étant égale au moins à 3, préparés conformément au procédé selon la revendication 1, dans des compositions alcalines contenant des solvants ou diluants, de l'eau de préférence, pour l'amélioration de l'effet mouillant des compositions.

3. Utilisation d'alcoolates exempts d'alcanols, de formule générale (I)
CₙH₂ₙ₊₁O(A)ₓ(B)_{y}R (I)
dans laquelle
R représente un groupe alkyle en C₁-C₆ ou benzyle
A représente un groupe éthylène-oxy
B représente un groupe alkylène(C₃-C₆)oxy ou des mélanges de tels groupes,
les groupes A et B pouvant être présents en distribution statistique, en alternance ou sous forme de deux ou plus de deux séquences en un ordre quelconque,
n représente un nombre entier allant de 4 à 8
x représente un nombre allant de 3 à 25
y représente un nombre allant de 0 à 10,
la somme x+y étant égale au moins à 3, préparés conformément au procédé selon la revendication 1, pour la diminution de la tension superficielle et l'accélération de l'ajustement de la tension superficielle dans des dispersions aqueuses ou compositions aqueuses de tensioactifs pour conférer une hydrophilie à des pigments ou des particules ou pour lier des pigments finement divisés et des poussières.

4. Agent mouillant, de nettoyage de métaux ou de dégraissage ou composition phytosanitaire, contenant un alcoolate exempt d'alcanol, de formule générale (I)
CₙH₂ₙ₊₁O(A)ₓ(B)_{y}R (I)
dans laquelle
R représente un groupe alkyle en C₁-C₆ ou benzyle
A représente un groupe éthylène-oxy
B représente un groupe alkylène(C₃-C₆)oxy ou des mélanges de tels groupes,
les groupes A et B pouvant être présents en distribution statistique, en alternance ou sous forme de deux ou plus de deux séquences en un ordre quelconque,
n représente un nombre entier allant de 4 à 8
x représente un nombre allant de 3 à 25
y représente un nombre allant de 0 à 10,
la somme x+y étant égale au moins à 3, préparé conformément au procédé selon la revendication 1.

5. Peintures, compositions pour des applications au pistolet, le traitement de minerais, l'industrie du bois et du papier ou pour des revêtements, des produits de revêtement, de colle, de traitement du cuir, de traitement de métaux, d'usinage de métaux ou de traitement de textiles, contenant un alcoolate exempt d'alcanol, de formule générale (I)
CₙH₂ₙ₊₁O(A)ₓ(B)_{y}R (I)
dans laquelle
R représente un groupe alkyle en C₁-C₆ ou benzyle
A représente un groupe éthylène-oxy
B représente un groupe alkylène(C₃-C₆)oxy ou des mélanges de tels groupes,
les groupes A et B pouvant être présents en distribution statistique, en alternance ou sous forme de deux ou plus de deux séquences en un ordre quelconque,
n représente un nombre entier allant de 4 à 8
x représente un nombre allant de 3 à 25
y représente un nombre allant de 0 à 10,
la somme x+y étant égale au moins à 3, préparé conformément au procédé selon la revendication 1.

6. Utilisation d'alcoolates exempts d'alcanols, de formule générale (I)
CₙH₂ₙ₊₁O(A)ₓ(B)_{y}R (I)
dans laquelle
R représente un groupe alkyle en C₁-C₆ ou benzyle
A représente un groupe éthylène-oxy
B représente un groupe alkylène(C₃-C₆)oxy ou des mélanges de tels groupes,
les groupes A et B pouvant être présents en distribution statistique, en alternance ou sous forme de deux ou plus de deux séquences en un ordre quelconque,
n représente un nombre entier allant de 4 à 8
x représente un nombre allant de 3 à 25
y représente un nombre allant de 0 à 10,
la somme x+y étant égale au moins à 3, préparés conformément au procédé selon la revendication 1, pour accroître la vitesse de mouillage dans des agents mouillants aqueux.

7. Composition de revêtement selon la revendication 5, **caractérisée en ce qu'**il s'agit d'une composition aqueuse qui contient de l'eau, des pigments, des liants et de 0,05 à 5 % en poids, par rapport aux pigments, d'un alcoolate exempt d'alcanol, de formule générale (I)
CₙH₂ₙ₊₁O(A)ₓ(B)_{y}R (I)
dans laquelle
R représente un groupe alkyle en C₁-C₆ ou benzyle
A représente un groupe éthylène-oxy
B représente un groupe alkylène(C₃-C₆)oxy ou des mélanges de tels groupes,
les groupes A et B pouvant être présents en distribution statistique, en alternance ou sous forme de deux ou plus de deux séquences en un ordre quelconque,
n représente un nombre entier allant de 4 à 8
x représente un nombre allant de 3 à 25
y représente un nombre allant de 0 à 10,
la somme x+y étant égale au moins à 3, préparé conformément au procédé selon la revendication 1.

8. Composition de revêtement selon la revendication 7, **caractérisée en ce qu'**il s'agit d'une composition pour des applications au pistolet.

9. Composition de peinture selon la revendication 5, **caractérisée en ce qu'**il s'agit d'une composition de peinture qui est appliquée par enduction au rideau ou enduction à la lame.
